# EUROPEAN PATENT SPECIFICATION

(11) **EP 0 275 515 B1**
(45) Date of publication and mention of the grant of the patent: **06.04.1994**
(21) Application number: 87118845.4
(22) Date of filing: 18.12.1987
(51) Int. Cl.: A61K 31/535

(54) **Aqueous pharmaceutical composition**
Wässeriges Arzneimittel
Composition pharmaceutique aqueuse

(30) Priority: 19.12.1986 JP 303100/86
(43) Date of publication of application: 27.07.1988
(73) Proprietor: DAIICHI PHARMACEUTICAL CO., LTD., Chuo-ku, Tokyo 103 (JP)
(72) Inventor: Seki, Hiromitsu, Daiichi Seiyaku Research Inst., Edogawa-ku, Tokyo (JP); Murata, Yukihiro, Daiichi Seiyaku Research Inst., Edogawa-ku, Tokyo (JP)
(74) Representative: Kinzebach, Werner, Dr.

(56) References cited:
- EP-A- 203 283
- EP-A- 0 142 426
- CHEMICAL ABSTRACTS, vol. 104, no. 15, 14 April 1986, Columbus, OH (US); I. HAYAKAWA et al., p. 382, no. 126370e#

## Description

The present invenion relates to an aqueous pharmaceutical composition for the treatment and prevention of infections and suppurative diseases in the form of an eye-drop, nasal drop or syrup, comprising an effective amount of S-(-)-9-fluoro-3-methyl-10-(4-methyl-1-piperazinyl)-7-oxo-2,3-dihydro-7H-pyrido[1,2,3-de][1,4]benzoxazine-6-carboxylic acid and an aqueous vehicle.

Racemic 9-fluoro-3-methyl-10-(4-methyl-1-piperazinyl)-7-oxo-2,3-dihydro-7H-pyrido-[1,2,3-de][1,4]benzoxazine-6-carboxylic acid is known as ofloxacin and exhibits antimicrobial activity. EP-A-142 426 discloses ophthalmic antibiotic formulations comprising an ophthalmologically acceptable carrier and an effective antibiotic amount of ofloxacin.

EP-A-206283 which is a prior art document in the sense of Art. 54(3) EPC discloses S-(-)-9-fluoro-3-methyl-10-(4-methyl-1-piperazinyl)-7-oxo-2,3-dihydro-7H-pyrido[1,2,3-de][1,4]benzoxazine-6-carboxylic acid (hereinafter referred to as the S-compound) as a quinolone derivative having excellent antimicrobial activity
The present inventors found that this optically active derivative is well soluble in water, remains stable in aqueous solution and, therefore, is useful in the form of an aqueous pharmaceutical composition.

Thus, it has now been confirmed that when a 0.5 w/v% aqueous solution of the S-compound in the pH range of 3 to 10 was stored at 60°C for 1 or 2 weeks and the residual concentrations were determined, the compound stably remained without decomposition.

This invention relates to an aqueous pharmaceutical composition for the treatment and prevention of infections and suppurative diseases in the form of an eye-drop, nasal drop or syrup, comprising an effective amount of S-(-)-9-fluoro-3-methyl-10-(4-methyl-1-piperazinyl)-7-oxo-2,3-dihydro-7H-pyrido[1,2,3-de][1,4]benzoxazine-6-carboxylic acid and an aqueous vehicle.

The aqueous pharmaceutical compositions according to the present invention are suited for the treatment and prevention of various infections and suppurative diseases including pneumonia, bronchitis, pharyngitis, laryngitis, amygdalitis, pyolitis, nephritis, cystitis, prostatitis, urethritis, folliculitis, hindradenitis, cholecystitis, cholangitis, otitis media, sinusitis, blepharitis, hordeolum, bacillary dysentery, enteritis, and the like.

The concentration of the S-compound in an aqueous pharmaceutical composition of the present invention is generally in the range of 0.01 to 20 w/v% and preferably in the range of 0.07 to 10 w/v%, although higher or lower concentrations may be employed depending on circumstances. The aqueous vehicle may be water, for example, distilled water, but various pharmaceutically acceptable additives may be incorporated as required.

Thus, such additives as various stabilizers, solubilizers, antioxidants, pH adjusting agents, dispersing agents, aromatics, soothing agents, flow controlling agents, surfactants, buffers, corrigents, colors, thickeners, solvents, sweeteners, emulsifiers, foaming agents, antifungal agents, solubilizers, suspending agents, perfumes, antifoams, isotonizing agents, preservatives, cosolvents, may be selectively employed according to the dosage form and intended application. Examples of such pharmaceutically acceptable additives include p-hydroxybenzoic esters, hydrochloric acid, sodium hydroxide, potassium chloride, sodium chloride, sodium carbonate, sodium hydrogencarbonate, citric acid, sodium citrate, acetic acid, sodium acetate, lactic acid, sodium lactate, succinic acid, sodium succinate, tartaric acid, sodium tartarate, phosphoric acid, sodium phosphate, potassium phosphate, sodium mono-hydrogenphosphate, sodium dihydrogenphosphate, potassium dihydrogenphosphate, trometanol, benzalkonium chloride, sugars such as sucrose, glucose, sorbitol, mannitol, etc., amino acids, alcohols, glycerol, carboxymethyl cellulose sodium and so on.

The following examples are further illustrative of the present invention.

### EXAMPLE 1

| Eye-drops or nasal drops: | |
|---|---|
| S-compound | 3 g |
| Sodium chloride | 9 g |
| Benzalkonium chloride (10 w/v%) | 0.25 mℓ |
| Sodium hydroxide | q.s. |
| Hydrochloric acid | q.s. |
| Purified water to make | 1,000 mℓ |

In approximately 950 mℓ of purified water were dissolved 3 g of the S-compound and 9 g of sodium chloride, followed by addition of 0.25 mℓ of benzalkonium chloride (10 w/v%). The mixture was adjusted to pH 6.0 to 7.5 with sufficient quantities of sodium hydroxide and hydrochloric acid, diluted with purified water to make 1,000 mℓ, and filtered. The eye-drops prepared in the above manner were suited for the treatment and prevention of infectious diseases of the eye and the recommended daily dosage of the eye-drops for an adult for this purpose was 0.005 to 1 mg and preferably 0.05 to 0.2 mg, to be instilled in a single dose or in divided doses. The nasal drops can be sprayed with a nebulizer nozzle into the nostrils for the treatment and prevention of intranasal infections.

### EXAMPLE 2

| Syrup: | |
|---|---|
| S-compound | 10 g |
| Sucrose | 400 g |
| Citric acid | 1.2 g |
| Sodium citrate | 7 g |
| Methyl p-hydroxybenzoate | 1.8 g |
| Propyl p-hydroxybenzoate | 0.2 g |
| Perfume | Trace |
| Pigment | Trace |
| Purified water to make | 1,000 mℓ |

In purified water were dissolved 10 g of the S-compound, 400 g of sucrose, 1.2 g of citric acid, 7 g of sodium citrate, 1.8 g of methyl p-hydroxybenzoate, 0.2 g of propyl p-hydroxybenzoate, and traces of perfume and pigment (selected from the Table (1) of Tar Pigments which can be used in pharmaceutical products, etc., under the Ministry of Health and Welfare Decree in Japan) to make 1,000 mℓ, and the solution was filtered.

This syrup was suited for the treatment and prevention of various infections and suppurative diseases including respiratory tract infections, urinary tract infections and surgical infections. The recommended daily dosage of S-compound in a syrup for adults was 50 to 600 mg and preferably 100 to 300 mg. The syrup is a particularly suitable dosage form for infants and children and the dosage for this application may be the adult dosage to about one-fifth thereof depending on age.

It is to be noted that the acute toxicity of the S-compound in mice (LD₅₀, intravenous) is 244 mg/kg.

## Claims (Claims for the following Contracting State(s): AT, BE, CH, DE, FR, GB, IT, LI, LU, NL, SE)

1. An aqueous pharmaceutical composition for the treatment and prevention of infections and suppurative diseases in the form of an eye-drop, nasal drop or syrup, comprising an effective amount of S-(-)-9-fluoro-3-methyl-10-(4-methyl-1-piperazinyl)-7-oxo-2,3-dihydro-7H-pyrido[1,2,3-de][1,4]benzoxazine-6-carboxylic acid and an aqueous vehicle.

## Claims (Claims for the following Contracting State(s): ES, GR)

1. A process for preparing an aqueous pharmaceutical composition for the treatment and prevention of infections and suppurative diseases in the form of an eye-drop, nasal drop or syrup, which comprises incorporating effective amount of S-(-)-9-fluoro-3-methyl-10-(4-methyl-1-piperazinyl)-7-oxo-2,3-dihydro-7H-pyrido[1,2,3-de][1,4]benzoxazine-6-carboxylic acid into an aqueous vehicle.

## Patentansprüche (Patentansprüche für folgende(n) Vertragsstaat(en): AT, BE, CH, DE, FR, GB, IT, LI, NL, SE)

1. Wäßriges pharmazeutisches Mittel zur Behandlung und Verhütung von Infektionen und suppurativen Krankheiten in Form von Augentropfen, Nasentropfen oder eines Sirups, umfassend eine wirksame Menge S-(-)-9-Fluoro-3-methyl-10-(4-methyl-1-piperazinyl)-7-oxo-2,3-dihydro-7H-pyrido[1,2,3-de][1,4]benzoxazin-6-carbonsäure und einen wäßrigen Träger.

## Patentansprüche (Patentansprüche für folgende(n) Vertragsstaat(en): ES, GR)

1. Verfahren zur Herstellung eines wäßrigen pharmazeutischen Mittels zur Behandlung und Verhütung von Infektionen und suppurativen Krankheiten in Form von Augentropfen, Nasentropfen oder eines Sirups, wobei man eine wirksame Menge S-(-)-9-Fluoro-3-methyl-10-(4-methyl-1-piperazinyl)-7-oxo-2,3-dihydro-7H-pyrido[1,2,3-de][1,4]benzoxazin-6-carbonsäure in einem wäßrigen Träger aufnimmt.

## Revendications (Revendications pour l'(les) Etat(s) contractant(s) suivant(s): AT, BE, CH, DE, FR, GB, IT, LI, NL, SE)

1. Composition pharmaceutique aqueuse pour le traitement et la prévention d'infections et d'affections suppurées sous la forme de collyres, gouttes nasales ou sirop, comprenant une quantité efficace d'acide S-(-)-9-fluoro-3-méthyl-10-(4-méthyl-1-pipérazinyl)-7-oxo-2,3-dihydro-7H-pyrido[1,2,3-de][1,4] benzoxazine-6-carboxylique et un véhicule aqueux.

## Revendications (Revendications pour l'(les) Etat(s) contractant(s) suivant(s): ES, GR)

1. Procédé pour la préparation d'une composition pharmaceutique aqueuse, pour le traitement et la prévention d'infections et d'affections suppurées, sous la forme de collyres, gouttes nasales ou sirop, comprenant l'incorporation d'une quantité efficace d'acide S-(-)-9-fluoro-3-méthyl-10-(4-méthyl-1-pipérazinyl)-7-oxo-2,3-dihydro-7H-pyrido[1,2,3-de][1,4] benzoxazine-6-carboxylique dans un véhicule aqueux.
